# FASCICULE DE BREVET EUROPEEN

(11) **EP 1 429 672 B1**
(45) Date de publication et mention de la délivrance du brevet: **17.01.2007**
(21) Numéro de dépôt: 02774928.2
(22) Date de dépôt: 25.09.2002
(51) Int. Cl.: A61B 17/70

(54) **DISPOSITIF DE FIXATION VERTEBRALE**
VORRICHTUNG ZUR WIRBELSÄULENFIXIERUNG
VERTEBRAL FIXING DEVICE

(30) Priorité: 26.09.2001 FR 0112354
(43) Date de publication de la demande: 23.06.2004
(73) Titulaire: Abbott Spine, La Cité Mondiale, 33000 Bordeaux (FR)
(72) Inventeur: MAZDA, Keyvan, F-75020 PARIS (FR); LE COUEDIC, Régis, F-78570 Andrésy (FR)
(74) Mandataire: Dronne, Guy
(86) Numéro de dépôt international: PCT/FR2002/003263
(87) Numéro de publication internationale: WO 2003/026521

(56) Documents cités:
- US-A- 4 003 376
- US-A- 5 928 232

## Description

La présente invention concerne un dispositif de fixation vertébrale susceptible d'être monté sur une vertèbre, et un ensemble de redressement du rachis utilisant un tel dispositif.

Un domaine d'application envisagé est notamment, mais non exclusivement, le traitement des scolioses ou, plus généralement des corrections de courbures anormales du rachis.

Le rachis est formé par la superposition de vertèbres, normalement alignées selon un axe vertébral, des lombaires jusqu'aux cervicales et chacune présentant une paroi postérieure de laquelle fait saillie l'apophyse épineuse et deux bords latéraux des parois desquels font saillies les côtes et/ou les apophyses transverses. Lorsque le rachis d'un individu présente une courbure anormale, les vertèbres sont inclinées les unes par rapport aux autres et par rapport audit axe vertébral. Les bords latéraux des vertèbres situés d'un même côté sont ainsi rapprochés les uns des autres et forment une concavité alors que les bords latéraux de l'autre côté apparaissent éloignés les uns des autres et forment une convexité.

Afin de redresser la colonne vertébrale, les bords latéraux des vertèbres du côté concave sont éloignés les uns des autres et portés, les uns par rapport aux autres à une distance sensiblement équivalente à celle qui sépare les bords latéraux de l'autre côté. Pour maintenir ensuite les vertèbres les unes par rapport aux autres, des dispositifs connus comprennent des vis que l'on insère dans les vertèbres ou des crochets que l'on introduit le long de la paroi interne du canal rachidien et des tiges destinées à relier les vis ou les crochets.

Les crochets sont généralement introduits deux par deux dans chaque vertèbre et de chaque côté à proximité des pédicules, leur tête faisant saillie de la paroi postérieure de la vertèbre, une de chaque côté de l'apophyse épineuse. Les têtes formant tulipe, par exemple, sont susceptibles de recevoir une tige qui est bloquée au moyen d'un écrou vissé sur la tête et en appui sur la tige. Les rangées constituées par les têtes de crochet situées de chaque côté des apophyses épineuses sont reliées entre elles et maintenues en position fixe par deux tiges parallèles entre elles et à l'axe du rachis.

Cependant, l'utilisation de ces crochets est délicate puisque l'opérateur ne doit en aucun cas affecter la moelle épinière qui s'étend au centre du canal rachidien, sous peine de provoquer une paralysie du patient.

L'utilisation des vis permet de diminuer les risques de l'intervention. Elles présentent également des têtes formant tulipe et sont insérées, deux par deux sur la paroi postérieure des vertèbres dans les pédicules de chaque côté de l'apophyse épineuse. Ainsi, les vis constituent des points de fixation dans les vertèbres pour les maintenir les unes par rapport aux autres. Cependant, elles sont nécessairement introduites dans le pédicule des vertèbres qui, dans certaines circonstances, est de faible taille ou détérioré.

Un dispositif de fixation vertébrale selon le préambule de la revendication 1 est connu du document US-A-5 928 232.

Un problème qui se pose et que vise à résoudre la présente invention est alors de constituer des points de fixation, lorsqu'il n'est pas possible d'introduire de vis dans les vertèbres de la portion incurvée et que l'utilisation des crochets s'avère trop dangereuse.

Pour atteindre ce but, selon un premier objet, la présente invention propose un dispositif de fixation vertébrale comprenant une pièce transversale, de forme allongée, présentant deux extrémités opposées sensiblement symétriques l'une de l'autre par rapport à un plan de symétrie coupant orthogonalement ladite pièce longitudinale transversale, chaque extrémité présentant une première face principale, une seconde face principale et une tranche, ladite pièce étant susceptible d'être disposée contre ladite paroi postérieure de ladite vertèbre sensiblement perpendiculairement à l'axe de ladite colonne vertébrale, chacune des premières faces principales desdites extrémités étant située en regard d'une côte et/ou d'une apophyse transverse ; au moins deux éléments de liaison réglables formant étriers, susceptibles d'être reliés respectivement à chacune desdites deux extrémités en regard de ladite première face principale et espacés l'un de l'autre, lesdites côtes et/ou apophyses transverses étant susceptibles d'être engagées dans lesdits éléments de liaison de façon à maintenir ladite pièce transversale en position fixe contre ladite paroi postérieure de ladite vertèbre ; et ledit dispositif comporte des moyens d'ancrage situés dans lesdites extrémités de ladite pièce transversale sur lesquels des organes de liaison sont susceptibles d'être montés, pour entraîner ladite vertèbre.

Ainsi, une caractéristique du dispositif de fixation vertébrale réside dans le mode de réalisation de points de fixation sur chacune des vertèbres du rachis au moyen de pièces transversales qui sont rendues solidaires de chaque vertèbre. Ces pièces transversales ne sont pas fixées sur les vertèbres au moyen de vis, mais grâce à deux éléments de liaison réglables pour chacune d'elles, qui relient leurs extrémités aux apophyses transverses et/ou aux côtes situées de chaque côté des vertèbres. De la sorte, les moyens d'ancrage sont susceptibles d'être montés sur les extrémités des pièces transversales solidaires d'une suite de vertèbres à déplacer, et ils sont susceptibles d'être maintenus en position fixe les uns par rapport aux autres au moyen de deux tiges, une de chaque côté des apophyses épineuses, et de systèmes de fixation qui les relient solidairement aux portions de tiges.

Selon un mode particulièrement avantageux de mise en oeuvre de l'invention, chacun desdits éléments de liaison réglables comporte une pièce en U, dont les extrémités libres des branches, espacées l'une de l'autre, sont munies de moyens de liaison et de moyens de réglage susceptibles de coopérer avec lesdites extrémités de ladite pièce transversale. De la sorte, pour chacun des deux côtés d'une vertèbre, la pièce en U est engagée sur l'apophyse transverse et/ou sur la côte qui fait saillie de la paroi du bord latéral de la vertèbre, de façon que le fond de la pièce en U prenne appui contre la paroi antérieure de l'apophyse ou de la côte et que les branches soient dirigées sensiblement dans la même direction que les apophyses épineuses. Les deux pièces en U sont ensuite reliées aux deux extrémités d'une pièce longitudinale transverse grâce aux moyens de liaison et aux moyens de réglage situés sur les extrémités libres des branches.

Selon un mode particulier de réalisation, ladite pièce en U est réalisée dans un matériau déformable susceptible de s'appliquer contre les contours desdites côtes et/ou apophyses transverses engagées dans ladite pièce en U. Ainsi, la pièce en U présente des points de contact sur l'ensemble du pourtour des côtes ou des apophyses et assure une meilleure solidarisation.

De façon préférentielle, chacune desdites extrémités de ladite pièce transversale présente au moins deux évidements débouchant respectivement, au moins dans lesdites première et seconde faces principales desdites extrémités et situés l'un par rapport à l'autre, sensiblement perpendiculairement à ladite pièce transversale, lesdits évidements étant susceptibles de coopérer avec lesdits éléments de liaison réglables. Ainsi, comme on l'expliquera plus en détail dans la suite de la description, les éléments de liaison réglables sont reliés de façon simplifiée à la pièce transversale grâce aux évidements qui traversent de part en part ses extrémités.

Selon un mode de réalisation de l'invention particulièrement avantageux, au moins un des deux évidements débouche dans la tranche de ladite extrémité dans la direction opposée à l'autre évidement et forme un trou oblong. Ainsi, comme on l'expliquera également plus en détail dans la suite de la description, cette disposition permet, dans un mode particulier de mise en oeuvre de l'invention, de réaliser un montage simplifié et rapide des pièces transversales sur les vertèbres.

Avantageusement, lesdites extrémités libres desdites branches de ladite pièce en U sont susceptibles d'être insérées librement dans lesdits évidements, en regard de ladite première face principale, lesdits moyens de liaison et lesdits moyens de réglage, solidaires desdites extrémités libres étant susceptibles de prendre appui contre ladite seconde face principale pour bloquer en translation ladite pièce en U dans la direction opposée à ladite première face principale et pour entraîner en translation au moins une desdites branches vers ladite première face principale. Ainsi, lorsque la pièce transversale est en appui contre la paroi postérieure de la vertèbre et que les deux pièces en U situées de chaque côté de la vertèbre, engagées derrière les apophyses transverses ou les côtes et en appui contre elles et que les extrémités libres des branches de chaque pièce en U sont insérées dans les évidements correspondants, la mise en oeuvre desdits moyens de réglage permet d'immobiliser ladite pièce longitudinale contre la vertèbre.

Selon un premier mode particulier de mise en oeuvre de l'invention, lesdits moyens de liaison sont formés par des moyens formant épaulement à l'extrémité libre de la branche de la pièce en U, l'extrémité libre de l'autre branche comportant les moyens de réglage. Ainsi, ledit épaulement est susceptible de venir en appui contre la seconde face principale de façon à bloquer en translation ladite branche dans la direction opposée à ladite première face principale, le fond de la pièce en U venant en appui contre la paroi antérieure de la côte ou de l'apophyse transverse, tandis que l'extrémité libre de l'autre branche comporte les moyens de réglage. De la sorte, l'actionnement des moyens de réglage permet d'entraîner la branche sur laquelle ils sont montés vers la première face principale, et fait pivoter la pièce en U autour du point d'appui de l'épaulement entraînant par là même, le fond de la pièce en U vers la première face principale et enserrant ainsi la côte et/ou l'apophyse. Bien évidemment, selon ce premier mode particulier de mise en oeuvre de l'invention, les deux branches doivent être suffisamment espacées l'une de l'autre pour obtenir du jeu entre la pièce en U et les côtes ou l'apophyse et permettre le pivotement sans gêne.

De façon particulièrement avantageuse, lesdites extrémités libres desdites branches présentent des moyens formant filetage sur lesquels un écrou est susceptible d'être vissé pour former lesdits moyens de liaison et lesdits moyens de réglage. Ainsi, l'écrou est susceptible d'être actionné en rotation autour de l'extrémité libre des branches, ce qui a pour effet, dans le mode de serrage, de le déplacer vers le fond de la pièce en U. De la sorte, l'écrou prenant appui contre la seconde face principale, sur le pourtour de l'évidement que l'extrémité libre de la branche traverse, la pièce en U est entraînée vers la première face principale opposée à la seconde.

Selon un mode particulier de réalisation de l'invention, lesdits moyens d'ancrage sont solidarisés à ladite seconde face principale desdites extrémités et font saillie sensiblement perpendiculairement à ladite seconde face principale. De la sorte, les moyens d'ancrage s'étendent dans une direction opposée à la paroi postérieure de la vertèbre dans un espace dégagé qu'un système de fixation est susceptible d'occuper.

Selon un autre mode particulier de réalisation de l'invention, lesdits moyens d'ancrage prolongent lesdits éléments de liaison réglables en regard de ladite seconde face principale. Ainsi, les moyens d'ancrage s'étendent également dans une direction opposée à la paroi postérieure de la vertèbre, mais ils sont solidaires des éléments de liaison réglable, ce qui diminue le nombre d'opérations de montage de la pièce longitudinale en elle-même et permet d'obtenir un dispositif de fixation vertébrale plus résistant à quantité de matière égale.

Selon un second objet, la présente invention propose un ensemble de redressement du rachis comprenant une pluralité de dispositifs de fixation vertébrale conformes à la présente invention montée sur une pluralité de vertèbres successives, lesdits moyens d'ancrage formant sensiblement deux alignements situés de chaque côté de la rangée des apophyses épineuses de ladite pluralité de vertèbres ; des systèmes de fixation montés sur chacun des moyens d'ancrage, susceptibles de recevoir une tige, chacun desdits dispositifs étant susceptible de maintenir le moyen d'ancrage et la tige en position fixe l'un par rapport à l'autre ; et, deux tiges longitudinales reliant respectivement lesdits dispositifs de chacun desdits alignements des moyens d'ancrage de chaque côté de ladite rangée des apophyses épineuses, de façon à maintenir en position fixe, l'un par rapport à l'autre au moins les points d'ancrage d'un même alignement.

D'autres particularités et avantages de l'invention ressortiront mieux à la lecture de la description faite ci-après de modes de réalisation particuliers de l'invention, donnés à titre indicatif mais non limitatif, en référence aux dessins annexés sur lesquels :
- la Figure 1 est une vue éclatée montrant un premier mode de réalisation d'un dispositif de fixation conforme à l'invention ;
- la Figure 2, est une vue en perspective montrant une portion de rachis comprenant deux vertèbres équipées de deux dispositifs de fixation vertébrale tels que représentés sur la Figure 1 ;
- la Figure 3 est une vue éclatée montrant un second mode de réalisation d'un dispositif de fixation vertébrale conforme à l'invention ; et,
- la Figure 4 est une vue en perspective montrant une portion de rachis comprenant deux vertèbres équipées de deux dispositifs de fixation vertébrale tels que représentés sur la Figure 3.

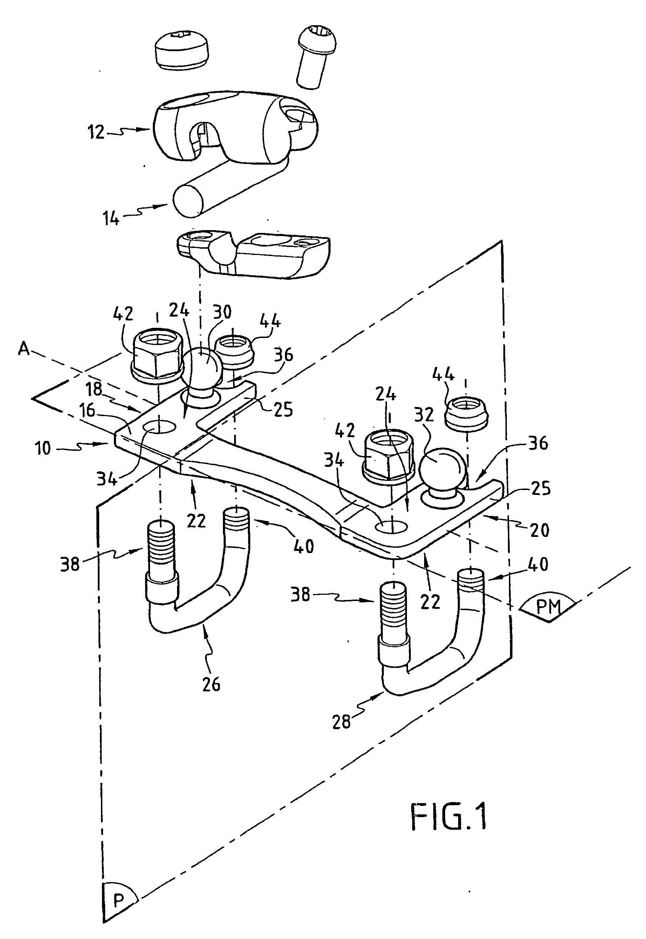

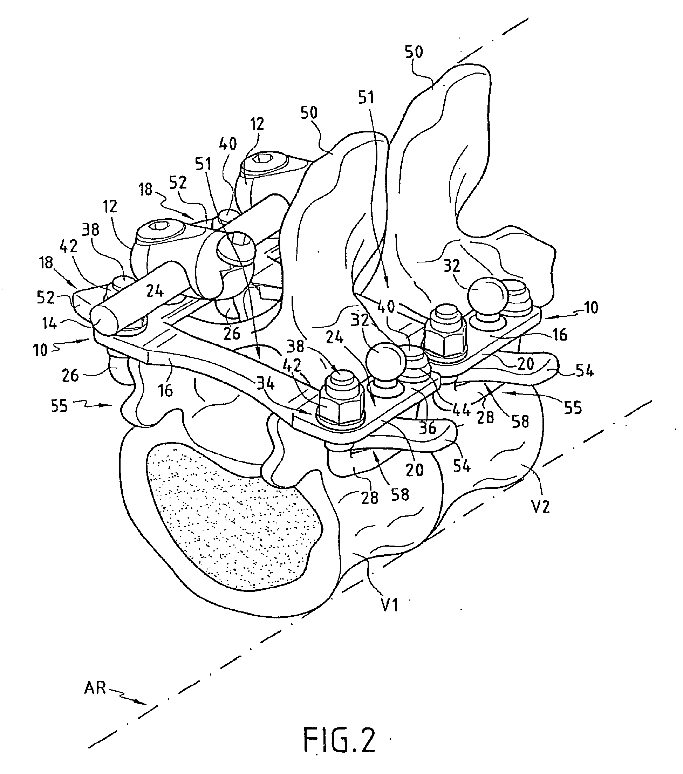

On se réfèrera aux Figures 1 et 2 pour décrire le dispositif de fixation vertébrale selon un premier mode de réalisation.

La Figure 1 montre un dispositif de fixation vertébrale 10 conforme à l'invention susceptible de recevoir au moins un système de fixation 12 permettant de maintenir en position fixe une tige 14 dont uniquement une portion est représentée sur la Figure.

Le dispositif de fixation vertébrale 10, comporte une pièce allongée transversale 16 d'axe A, définissant un plan moyen Pm, et présentant deux extrémités opposées 18, 20. Ces deux extrémités 18, 20 sont sensiblement l'image l'une de l'autre par rapport à un plan de symétrie P qui coupe la pièce transversale 16 orthogonalement ; elles présentent chacune une première face principale 22 et une seconde face principale 24, ainsi qu'une tranche 25. Au regard des deux faces principales 22, deux éléments de liaison réglables 26 et 28, sont susceptibles d'être reliés respectivement aux extrémités 18 et 20. En outre, les extrémités comportent deux moyens d'ancrage 30 et 32 de forme sphérique faisant saillie de la seconde face principale 24.

Afin de réaliser cette liaison, les extrémités 18 et 20 présentent chacune, un premier évidement 34 formé d'un perçage et un second évidement 36 débouchant dans la face de tranche 25 dans la direction opposée au premier évidement 34. Les premier et second évidements sont espacés l'un de l'autre d'une première distance et traversent de part en part la pièce transversale 16, de façon à déboucher dans la première 22 et seconde 24 face principale. En outre, les premier 34 et second 36 perçages définissent une ligne, sensiblement perpendiculaire à la pièce transversale 16.

Les éléments de liaison 26 et 28 présentent la forme d'une pièce en U dont les extrémités libres 38 et 40 des deux branches, espacées l'une de l'autre d'une distance correspondant à ladite première distance, sont filetées de manière à pouvoir recevoir des écrous 42 et 44. On comprend que les extrémités libres 38 et 40 des branches sont destinées à être introduites dans les évidements 34 et 36, les éléments de liaisons 26 et 28 venant en regard, respectivement, des premières faces principales 22 de la pièce transversale 16. Lorsque ces extrémités libres 38 et 40, après avoir été insérées dans les évidements 34 et 36, font saillie de la seconde face principale 24, les écrous 42 et 44 sont susceptibles d'être vissés dessus pour retenir les pièces de liaison et comme on le décrira plus en détails dans la suite de la description, pour immobiliser la pièce transversale 16 contre la paroi postérieure de la vertèbre.

Les facilités de montage des éléments de liaison 26 et 28 sur la pièce transversale sont accrues, et plus particulièrement la vitesse de montage, grâce à la forme du second évidement 36, qui débouche également dans la face de tranche 25 et qui forme un trou oblong. Ainsi, lorsque la pièce transversale 16 est en position d'installation, les écrous 44 sont pré-montés sur les extrémités libres 40 des branches de la pièce en U et cette dernière est présentée dans un plan parallèle au plan P, l'extrémité libre 40 étant dirigée vers le second évidement 36 de façon que les branches soient inclinées par rapport au plan moyen Pm. Ensuite, la branche de la pièce en U présentant l'extrémité libre 40 est engagée, sensiblement parallèlement au plan moyen Pm, dans l'évidement 36 de façon que l'écrou 44 vienne en appui contre la seconde face 24. De la sorte, la pièce en U est susceptible d'être entraînée en pivotement autour du point d'appui de l'écrou 44 sur la seconde face, de façon que l'autre extrémité libre 38, initialement située au regard de la première face 22, vienne s'engager dans l'évidement 34 pour être ensuite maintenu par l'écrou 42. Comme on l'expliquera dans la suite de la description, lorsque la pièce en U pivote, elle est susceptible d'emprisonner une côte ou une apophyse située au regard de la première face 22.

Selon un mode particulier de réalisation de l'invention, la pièce en U est déformable de sorte qu'elle s'applique parfaitement contre le pourtour des objets qu'elle enserre, assurant ainsi une meilleure solidarisation. Les matériaux utilisés pour réaliser la pièce en U, selon ce mode de réalisation, sont essentiellement des polymères du type polyéthylène ou tout autre matériau biocompatible, sous une forme élaborée en tresse ou sous une forme brute.

Bien évidemment, les extrémités libres des branches de la pièce en U déformable doivent comporter des moyens de liaison et des moyens de réglage adaptés au matériau. Ainsi, les extrémités libres des branches sont serties dans un élément en matériau rigide, susceptible d'être fileté ou de constituer un épaulement. Selon un autre mode de mise en oeuvre, l'élément est susceptible d'être surmoulé sur l'extrémité libre des branches.

De la sorte, les moyens de liaison et les moyens de réglage sont solidarisés de façon irréversible aux extrémités libres des branches de la pièce en U de telle sorte qu'une traction prolongée exercée sur la pièce en U par l'intermédiaire des moyens de liaison et des moyens de réglage n'entraîne pas leur désolidarisation.

Les moyens d'ancrage 30 et 32 formés par des têtes sphériques sont situées sur la seconde surface principale 24, entre les deux évidements 34 et 36. Ils sont solidement reliés à la pièce transversale 16, par exemple par soudure, de façon que l'action mécanique qui est exercée sur lesdits moyens 30 et 32, puisse entraîner ladite pièce 16 sans désolidarisation.

Comme on va l'expliquer, en référence à la Figure 2, cette action mécanique est susceptible d'être effectuée au moyen d'un système de fixation 12 et d'une tige 14.

La Figure 2 montre deux vertèbres consécutives V1, V2, formant une portion de rachis et comportant chacune une apophyse épineuse 50 faisant saillie d'une paroi postérieure 51 et deux apophyses transverses 52 et 54 faisant saillie des parois latérales 55. Les vertèbres V1 et V2 sont chacune équipée d'un dispositif de fixation vertébrale 10 conforme à l'invention et tel que représenté sur la Figure 1. En outre, les deux dispositifs de fixation vertébrale 10 sont reliés entre eux au moyen de deux systèmes de fixation 12, lesquels sont également reliés entre eux par la tige 14.

Pour des raisons de clarté, deux vertèbres V1 et V2 uniquement sont représentées. Cependant, des dispositifs de fixation vertébrale sont susceptibles d'être montés sur une pluralité de vertèbres successives. De la même façon, une seule tige 14 et uniquement deux systèmes de fixation sont représentés alors que selon une configuration normale, des systèmes de fixation sont montés sur les têtes sphériques 32 de façon symétrique par rapport aux apophyses épineuses 50 et ils sont également reliés au moyen d'une tige.

Comme le montre la Figure 2, les pièces transversales 16 sont en appui contre la paroi postérieure 51 des vertèbres sensiblement perpendiculairement à l'axe AR du rachis. Elles sont maintenues dans cette position grâce aux pièces en U 28 et 26 dont les extrémités libres 38 et 40 sont immobilisées dans les évidements 34 et 36 au moyen des écrous 42 et 44 qui sont en appui contre la seconde face principale 24. On comprend que le serrage des écrous 42 et 44 autour des extrémités libres des branches entraîne le fond de la pièce en U contre la paroi antérieure 58 des apophyses transverses et par conséquent, l'entraînement des extrémités 18 et 20 des pièces transversales vers les apophyses transverses 54 et 52. De la sorte, la pièce allongée transversale 16 est maintenue de chaque côté de la vertèbre et s'appuie contre sa paroi postérieure. Ainsi, elle est rendue complètement solidaire de la vertèbre. Bien évidemment le serrage des écrous doit être suffisant et comprimer légèrement la paroi osseuse pour assurer un bon maintien.

Lorsque les dispositifs de fixation vertébrale sont solidaires des vertèbres, les systèmes de fixation équipés d'une tige sont montés et serrés sur les têtes sphériques, pour chacun des côtés du rachis, après que celui-ci a été éventuellement redressé. Ainsi les systèmes de fixation 12 sont maintenus en position fixe par rapport aux têtes sphériques et la tige est immobilisée par rapport aux systèmes de fixation, de sorte que les têtes sphériques sont immobiles les unes par rapport aux autres. En conséquence les vertèbres sont maintenues latéralement en position fixe les unes par rapport aux autres.

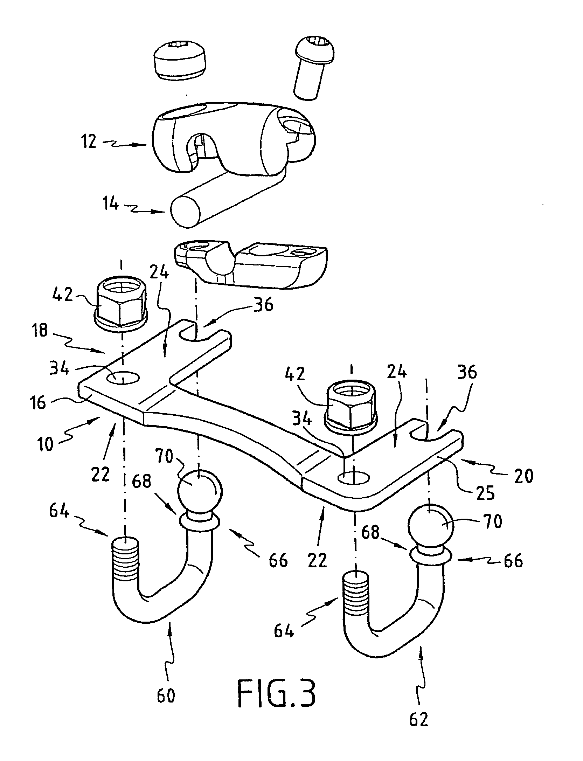

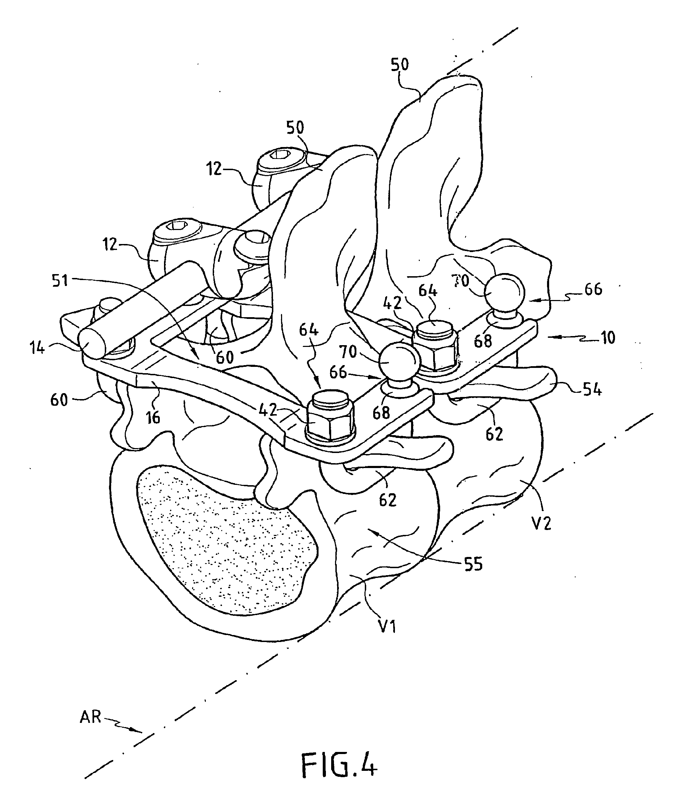

On se référera à la Figure 3 et à la Figure 4 pour décrire des éléments de liaison réglables formant étrier, selon un mode particulier de mise en oeuvre dans lequel les moyens d'ancrage sont reliés.

Selon ce mode de mise en oeuvre, les pièces en U, 60 et 62 présentent une extrémité libre 64 qui est filetée de façon à recevoir l'écrou 42 comme dans le mode de réalisation précédent ; en revanche, l'autre extrémité libre 66 se prolonge par un épaulement 68 surmonté d'une tête sphérique 70 formant les moyens d'ancrage. Ainsi, il n'est nul besoin d'équiper la pièce transversale de moyens d'ancrage.

De plus, tout comme pour la pièce en U du mode de réalisation précédent, pour lequel l'écrou est pré-monté sur l'extrémité libre de la branche de la pièce, l'extrémité libre 66 des pièces en U 60 et 62 est engagée dans l'évidement 36 de manière à ce que l'épaulement 68 soit en appui contre la seconde face principale 24 sur les pourtours de l'évidement 36. Ainsi, les pièces en U 60 et 62 sont susceptibles d'être entraînées à pivotement autour du point d'appui de l'épaulement 68 autour de l'évidement 36 contre la seconde surface principale 24, de façon que l'autre extrémité 64 de la pièce en U soit insérée dans l'évidement 34. De la sorte, lorsque l'écrou 42 est vissé sur l'extrémité libre 64 de la branche des pièces en U, 60 et 62, ces dernières sont bloquées en translation dans la direction opposée à la première face principale 22.

Ainsi, de façon avantageuse, la tête sphérique qui constitue les moyens d'ancrage est montée sur la pièce transversale 16 avec les éléments de liaison avec lesquels elle ne forme qu'une seule pièce. Outre la simplification de montage de la pièce transversale 16 en elle-même, que ce moyen de mise en oeuvre procure, les moyens d'ancrage sont plus fermement solidaires des vertèbres qu'ils vont permettre d'entraîner.

Dans les modes de réalisation précédemment décrits en référence aux Figures 2 et 3, les éléments de liaison sont engagés uniquement sur les apophyses transverses car les vertèbres, illustrées sur ces Figures, ne comportent pas de côte. Bien évidemment, on ne sortirait pas du cadre de l'invention, en montant une pluralité de dispositifs de fixation vertébrale, sur une pluralité de vertèbres auxquelles des côtes sont raccordées. C'est notamment le cas de vertèbres dorsales, dont les côtes se raccordent sur la paroi latérale sensiblement en regard de la paroi antérieure des apophyses transverses. Ainsi, les pièces en U, dont la longueur des branches est supérieure à celle des pièces en U utilisées pour les vertèbres dépourvues de côte, sont engagées sur les côtes de façon que le fond de la pièce soit en appui contre la paroi antérieure de la côte et que, tout comme dans les précédents modes de réalisation, les extrémités libres des branches traversent les évidements de la pièce transversale pour y être reliées. De la sorte, on comprend que les branches de la pièce en U se prolongent de chaque côté de l'apophyse transverse, mais qu'elles ne constituent plus un élément d'accrochage du dispositif de fixation vertébrale.

On ne sortirait pas non plus du cadre de l'invention, en utilisant des pièces en U en matériau déformable pour relier les pièces allongées transversales aux vertèbres par l'intermédiaire des côtes. De la même manière, la longueur de la pièce en U en matériau déformable serait augmentée.

## Revendications

1. Dispositif de fixation vertébrale susceptible d'être monté sur une vertèbre du rachis, ladite vertèbre présentant une paroi postérieure de laquelle fait saillie l'apophyse épineuse et deux parois latérales desquelles font saillies les côtes et/ou les apophyses transverses,
**caractérisé en ce qu'**il comprend :
- une pièce transversale (16) de forme allongée présentant deux extrémités opposées (18, 20) sensiblement symétriques l'une de l'autre par rapport à un plan de symétrie (P) coupant orthogonalement ladite pièce longitudinale transversale (16), chaque extrémité (18, 20) présentant une première face principale (22), une seconde face principale (24) et une tranche (25), ladite pièce transversale (16) étant susceptible d'être disposée contre ladite paroi postérieure (51) de ladite vertèbre sensiblement perpendiculairement à l'axe (AR) dudit rachis, chacune des premières faces principales (22) desdites extrémités (18, 20) étant située en regard d'une côte et/ou d'une apophyse transverse (52, 54);
- au moins deux éléments de liaison réglables (26, 28, 60, 62, 42, 44, 68) formant étriers, susceptibles d'être reliés respectivement à chacune desdites deux extrémités (18, 20) en regard de ladite première face principale (22) et espacés l'un de l'autre, lesdites côtes et/ou apophyses transverses (52, 54) étant susceptibles d'être engagées dans lesdits éléments de liaison réglables (26, 28 60, 62, 42, 44, 68) de façon à maintenir ladite pièce transversale (16) en position fixe contre ladite paroi postérieure (51) de ladite vertèbre ;
et **en ce que** ledit dispositif comporte des moyens d'ancrage (30, 32) situés dans lesdites extrémités (18, 20) de ladite pièce transversale (16) sur lesquels des organes de liaison (26, 28, 60, 62, 42, 44, 68) sont susceptibles d'être montés, pour entraîner ladite vertèbre.

2. Dispositif de fixation vertébrale selon la revendication 1, **caractérisé en ce que** chacun desdits éléments de liaison réglables (26, 28 60, 62, 42, 44, 68) comporte une pièce en U, dont les extrémités libres (38, 40, 64, 66) des branches, espacées l'une de l'autre, sont munies de moyens de liaison (42, 44, 68) et de moyens de réglage (42, 44) susceptibles de coopérer avec lesdites extrémités (18, 20) de ladite pièce transversale (16).

3. Dispositif de fixation vertébrale selon la revendication 2, **caractérisé en ce que** ladite pièce en U est réalisée dans un matériau déformable susceptible de s'appliquer contre les contours desdites côtes et/ou apophyses transverses (52, 54) engagées dans ladite pièce en U.

4. Dispositif de fixation vertébrale selon l'une quelconque des revendications 1 à 3, **caractérisé en ce que** chacune desdites extrémités (18, 20) de ladite pièce transversale (16) présente au moins deux évidements (34, 36) débouchant respectivement au moins dans lesdites première et seconde face principale (22, 24) desdites extrémités (18, 20) et situés l'un par rapport à l'autre, sensiblement perpendiculairement à ladite pièce transversale (16), lesdits évidements (34, 36) étant susceptibles de coopérer avec lesdits éléments de liaison réglables (26, 28 60, 62, 42, 44, 68).

5. Dispositif de fixation vertébrale selon la revendication 4, **caractérisé en ce qu'**au moins un des deux évidements (36) débouche dans la tranche (25) de ladite extrémité (18, 20) dans la direction opposée à l'autre évidement (34).

6. Dispositif de fixation vertébrale selon les revendications 2 ou 3 et 4 ou 5, **caractérisé en ce que** lesdites extrémités libres desdites branches (38, 40, 64, 66) de ladite pièce en U sont susceptibles d'être insérées librement dans lesdits évidements (34, 36), en regard de ladite première face principale (22), lesdits moyens de liaison (26, 28 60, 62, 42, 44, 68) et lesdits moyens de réglage (42, 44), solidaires desdites extrémités libres (38, 40, 64, 66) étant susceptibles de prendre appui contre ladite seconde face principale (24) pour bloquer en translation ladite pièce en U dans la direction opposée à ladite première face principale (22) et pour entraîner en translation au moins une desdites branches vers ladite première face principale (22).

7. Dispositif de fixation vertébrale selon la revendication 6, **caractérisé en ce que** lesdits moyens de liaison sont formés par des moyens formant épaulement (68) à l'extrémité libre (66) de la branche de la pièce en U.

8. Dispositif de fixation vertébrale selon la revendication 6, **caractérisé en ce que** lesdites extrémités libres (38, 40, 64) desdites branches présentent des moyens formant filetage sur lequel un écrou (42, 44) est susceptible d'être vissé pour former lesdits moyens de liaison et lesdits moyens de réglage (26, 28 60, 62, 42, 44, 68).

9. Dispositif de fixation vertébrale selon l'une quelconque des revendications 1 à 7, **caractérisé en ce que** lesdits moyens d'ancrage (30, 32) sont solidarisés à ladite seconde face principale (24) desdites extrémités (18, 20) et font saillie sensiblement perpendiculairement à ladite seconde face principale (24).

10. Dispositif de fixation vertébrale selon l'une quelconque des revendications 1 à 7, **caractérisé en ce que** lesdits moyens d'ancrage (70) prolongent lesdits éléments de liaison réglables (60, 62) en regard de ladite seconde face principale (24).

11. Ensemble de redressement du rachis, **caractérisé en ce qu'**il comprend :
- une pluralité de dispositifs de fixation vertébrale (10) selon l'une quelconque des revendications 1 à 9, montée sur une pluralité de vertèbres (V1, V2) successives, lesdits moyens d'ancrage formant sensiblement deux alignements situés de chaque côté de la rangée des apophyses épineuses (50) de ladite pluralité de vertèbres (V1, V2) ;
- des systèmes de fixation (12) montés sur chacun des moyens d'ancrage (30, 32, 70), susceptibles de recevoir une tige (14), chacun desdits dispositifs (12) étant susceptible de maintenir le moyen d'ancrage (30, 32, 70) et la tige (14) en position fixe l'un par rapport à l'autre ; et,
- deux tiges longitudinales (14) reliant respectivement lesdits dispositifs (12) de chacun desdits alignements des moyens d'ancrage (30, 32, 70) de chaque côté de ladite rangée des apophyses épineuses (50), de façon à maintenir en position fixe, l'un par rapport à l'autre au moins, les points d'ancrage (30, 32, 70) d'un même alignement.

## Claims

1. A vertebral fixing device suitable for being mounted on a vertebra of the spine, said vertebra presenting a posterior wall from which there projects a spinous process and two side walls from which there project ribs and/or transverse processes,
the device being **characterised in that** it comprises:
- a transverse part (16) of elongate shape presenting two opposite ends (18, 20) that are substantially symmetrical to each other about a plane of symmetry (P) intersecting said elongate transverse part (16) orthogonally, each end (18, 20) presenting a first main face (22), a second main face (24), and an end edge face (25), said transverse part (16) being suitable for being placed against said posterior wall (51) of said vertebra substantially perpendicularly to the axis (AR) of said spine, each of the first main faces (22) of said ends (18, 20) being situated facing a rib and/or a transverse process (52, 54);
- at least two adjustable connection elements (26, 28, 60, 62, 42, 44, 68) forming clamps, suitable for being connected respectively to each of said two ends (18, 20) facing said first main face (22) and spaced apart from each other, said ribs and/or transverse processes (52, 54) being suitable for being engaged in said adjustable connection elements (26, 28, 60, 62, 42, 44, 68) in such a manner as to hold said transverse part (16) in a fixed position against said posterior wall (51) of said vertebra; and
**in that** said device includes anchor means (30, 32) situated in said ends (18, 20) of said transverse part (16) on which the connection members (26, 28, 60, 62, 42, 44, 68) are suitable for being mounted in order to displace said vertebra.

2. A vertebral fixing device according to claim 1, **characterised in that** each of said adjustable connection elements (26, 28, 60, 62, 42, 44, 68) comprises a U-shaped part whose spaced-apart limbs have free ends (38, 40, 64, 66) that are provided with connection means (42, 44, 68) and adjustment means (42, 44) suitable for co-operating with said ends (18, 20) of said transverse part (16).

3. A vertebral fixing device according to claim 2, **characterised in that** said U-shaped part is made of a deformable material suitable for pressing against the outlines of said ribs and/or transverse processes (52, 54) engaged in said U-shaped part.

4. A vertebral fixing device according to any one of claims 1 to 3, **characterised in that** each of said ends (18, 20) of said transverse part (16) presents at least two recesses (34, 36) opening out respectively at least into said first and second main faces (22, 24) of said ends (18, 20) and situated relative to each other substantially perpendicularly to said transverse part (16), said recesses (34, 36) being suitable for co-operating with adjustable connection elements (26, 28, 60, 62, 42, 44, 68).

5. A vertebral fixing device according to claim 4, **characterised in that** at least one of the two recesses (36) opens out into the end edge face (25) of said end (18, 20) in the direction facing away from the other recess (34).

6. A vertebral fixing device according to claim 2 or claim 3 and claim 4 or claim 5, **characterised in that** said free ends of said limbs (38, 40, 64, 66) of said U-shaped part are suitable for being inserted freely in said recesses (34, 36) facing said first main face (22), said connection means (26, 28, 60, 62, 42, 44, 68) and said adjustment means (42, 44) secured to said free ends (38, 40, 64, 66) being suitable for bearing against said second main face (24) to prevent said U-shaped part from moving in translation away from said first main face (22) and for moving at least one of said limbs in translation towards said first main face (22).

7. A vertebral fixing device according to claim 6, **characterised in that** said connection means are formed by shoulder-forming means (68) at the free end (66) of the limb of the U-shaped part.

8. A vertebral fixing device according to claim 6, **characterised in that** said free ends (38, 40, 64) of said limbs present thread-forming means suitable for having a nut (42, 44) screwed thereon to form said connection means and said adjustment means (26, 28, 60, 62, 42, 44, 68).

9. A vertebral fixing device according to any one of claims 1 to 7, **characterised in that** said anchor means (30, 32) are secured to said second main faces (24) of said ends (18, 20) and project substantially perpendicularly to said second main faces (24).

10. A vertebral fixing device according to any one of claims 1 to 7, **characterised in that** said anchor means (70) extend said adjustable connection elements (60, 62) where they face said second main faces (24).

11. An assembly for straightening the spine, the assembly being **characterised in that** it comprises:
- a plurality of vertebral fixing devices (10) according to any one of claims 1 to 9 mounted on a plurality of successive vertebrae (V1, V2), said anchor means forming substantially two rows situated on either side of the row of spinous processes (50) of said plurality of vertebrae (V1, V2);
- fixing systems (12) mounted on each of the anchor means (30, 32, 70) and suitable for receiving a rod (14), each of said devices (12) being suitable for holding the anchor means (30, 32, 70) and the rod (14) in a fixed position relative to each other; and
- two longitudinal rods (14) interconnecting said devices (12) in each of said rows respectively of the anchor means (30, 32, 70) on either side of said row of spinous processes (50), thereby holding the anchor points (30, 32, 70) of a given row in fixed positions, at least relative to one another.

## Patentansprüche

1. Vorrichtung zur Wirbelsäulenfixierung, die auf einen Wirbel der Wirbelsäule montiert zu werden vermag, wobei der Wirbel eine Rückwand, von welcher der Dornfortsatz vorsteht, und zwei Seitenwände aufweist, von denen die Rippen und/oder die Querfortsätze vorstehen,
**dadurch gekennzeichnet, dass** sie umfasst:
- ein quer liegendes Teil (16) länglicher Form, das zwei gegenüber liegende Enden (18, 20) aufweist, die bezüglich einer das quer liegende Längsteil (16) orthogonal schneidenden Symmetrieebene (P) zueinander im Wesentlichen symmetrisch sind, wobei jedes Ende (18, 20) eine erste Hauptseite (22), eine zweite Hauptseite (24) und eine Schmalseite (25) aufweist, wobei das quer liegende Teil (16) gegen die Rückwand (51) des im Wesentlichen senkrecht zur Achse (AR) der Wirbelsäule verlaufenden Wirbels angeordnet zu werden vermag, wobei jede der ersten Hauptseiten (22) der Enden (18, 20) einer Rippe und/oder einem Querfortsatz (52, 54) gegenüber liegt;
- wenigstens zwei verstellbare, Bügel bildende Verbindungselemente (26, 28, 60, 62, 42, 44, 68), die jeweils mit jedem der beiden Enden (18, 20) gegenüber der ersten Hauptfläche (22) verbunden zu werden vermögen und voneinander beabstandet sind, wobei die Rippen und/oder Querfortsätze (52, 54) in die verstellbaren Verbindungselemente (26, 28, 60, 62, 42, 44, 68) derart eingeführt zu werden vermögen, dass das quer liegende Teil (16) in einer festen Lage gegen die Rückwand (51) des Wirbels gehalten wird;
und dass die Vorrichtung Verankerungsmittel (30, 32) umfasst, die in den Enden des quer liegenden Teils (16) angeordnet sind, auf denen die Verbindungseinrichtungen (26, 28, 60, 42, 44, 68) montiert zu werden vermögen, um den Wirbel zu führen.

2. Vorrichtung zur Wirbelsäulenfixierung nach Anspruch 1,
**dadurch gekennzeichnet, dass** jedes der verstellbaren Verbindungselemente (26, 28, 60, 62, 42, 44, 68) ein U-förmiges Teil aufweist, dessen voneinander beabstandete freie Enden (38, 40, 64, 66) der Schenkel mit Verbindungsmittein (42, 44, 68) und Einstellmitteln (42, 44) versehen sind, die mit den Enden (18, 20) des quer liegenden Teils (16) zusammenzuwirken vermögen.

3. Vorrichtung zur Wirbelsäulenfixierung nach Anspruch 2,
**dadurch gekennzeichnet, dass** das U-förmige Teil aus einem verformbaren Material ausgeführt ist, das gegen die Konturen der Rippen und/oder Querfortsätze (52, 54), die in das U-förmige Teil eingeführt sind, zu drücken vermag.

4. Vorrichtung zur Wirbelsäulenfixierung nach einem der Ansprüche 1 bis 3,
**dadurch gekennzeichnet, dass** jedes der Enden (18, 20) des quer liegenden Teils (16) wenigstens zwei Aussparungen (34, 36) aufweist, die wenigstens in die erste bzw. die zweite Hauptseite (22, 24) der Enden (18, 20) münden und bezüglich einander im Wesentlichen senkrecht zu dem quer liegenden Teil (16) angeordnet sind, wobei die Aussparungen (34, 36) mit den verstellbaren Verbindungselementen (26, 28, 60, 62, 42, 44, 68) zusammenzuwirken vermögen.

5. Vorrichtung zur Wirbelsäulenfixierung nach Anspruch 4,
**dadurch gekennzeichnet, dass** wenigstens eine der beiden Aussparungen (36) in entgegengesetzter Richtung zu der anderen Aussparung (34) in die Schmalseite (25) des Endes (18, 20) mündet.

6. Vorrichtung zur Wirbelsäulenfixierung nach den Ansprüchen 2 oder 3 und 4 oder 5,
**dadurch gekennzeichnet, dass** die freien Enden der Schenkel (38, 40, 64, 66) des U-förmigen Teils ungehindert in die Aussparungen (34, 36) gegenüber der ersten Hauptseite (22) eingeführt zu werden vermögen, wobei die Verbindungsmittel (26, 28, 60, 62, 42, 44, 68) und die Einstellmittel (42, 44), die mit den freien Enden (38, 40, 64, 66) fest verbunden sind, gegen die zweite Hauptseite (24) zur Anlage zu kommen vermögen, um das U-förmige Teil in entgegengesetzter Richtung zur ersten Hauptseite (22) translatorisch zu blockieren und um wenigstens einen der Schenkel zur ersten Hauptseite (22) translatorisch zu bewegen.

7. Vorrichtung zur Wirbelsäulenfixierung nach Anspruch 6,
**dadurch gekennzeichnet, dass** die Verbindungsmittel durch Mittel gebildet sind, die einen Absatz (68) an dem freien Ende (66) des Schenkels des U-förmigen Teils bilden.

8. Vorrichtung zur Wirbelsäulenfixierung nach Anspruch 6,
**dadurch gekennzeichnet, dass** die freien Enden (38, 40, 64) der Schenkel Mittel aufweisen, die ein Gewinde bilden, auf dem eine Mutter (42, 44) aufgeschraubt zu werden vermag, um die Verbindungs- und die Einstellmittel (26, 28, 60, 42, 44, 68) zu bilden.

9. Vorrichtung zur Wirbelsäulenfixierung nach einem der Ansprüche 1 bis 7,
**dadurch gekennzeichnet, dass** die Verankerungsmittel (30, 32) mit der zweiten Hauptseite (24) der Enden (18, 20) fest verbunden sind und im Wesentlichen senkrecht zu der zweiten Hauptseite (24) vorstehen.

10. Vorrichtung zur Wirbelsäulenfixierung nach einem der Ansprüche 1 bis 7,
**dadurch gekennzeichnet, dass** die Verankerungsmittel (70) die verstellbaren Verbindungselemente (60, 62) gegenüber der zweiten Hauptseite (24) verlängern.

11. Einheit zum Aufrichten der Wirbelsäule, **dadurch gekennzeichnet, dass** sie umfasst:
- eine Vielzahl von Vorrichtungen zur Wirbelsäulenfixierung (10) nach einem der Ansprüche 1 bis 9, die auf einer Vielzahl von aufeinander folgenden Wirbeln (V1, V2) montiert sind, wobei die Verankerungsmittel im Wesentlichen zwei fluchtend angeordnete Reihen bilden, die zu beiden Seiten der Reihe von Dornfortsätzen (50) der Vielzahl von Wirbeln (V1, V2) angeordnet sind;
- Fixierungssysteme (12), die auf jedem der Verankerungsmittel (30, 32, 70) angebracht sind und eine Stange (14) aufzunehmen vermögen, wobei jede dieser Vorrichtungen (12) das Verankerungsmittel (30, 32, 70) und die Stange (14) bezüglich einander feststehend zu halten vermögen; und
- zwei Längsstangen (14), welche jeweils die Vorrichtungen (12) jeder der fluchtend angeordneten Reihen von Verankerungsmitteln (30, 32, 70) auf jeder Seite der Reihe von Dornfortsätzen (50) derart miteinander verbinden, dass die Verankerungspunkte (30, 32, 70) einer fluchtend angeordneten Reihe wenigstens bezüglich einander feststehend gehalten werden.
